Europäisches Patentamt

European Patent Office

Office européen des brevets

(19)

(11) Numéro de publication: **0 230 393**
**A1**

## DEMANDE DE BREVET EUROPEEN

(12)

(21) Numéro de dépôt: 87430001.5

(22) Date de dépôt: **05.01.87**

(51) Int. Cl.⁴: **A 61 M 21/00**
**A 61 N 5/00**

(30) Priorité: **07.01.86 FR 8600240**
**21.07.86 FR 8610651**

(43) Date de publication de la demande:
**29.07.87 Bulletin 87/31**

(84) Etats contractants désignés:
**AT BE CH DE ES GB GR IT LI LU NL SE**

(71) Demandeur: **Camus, Patrick**
**315 rue Jean Jaurès**
**F-83600 Frejus (FR)**

**Sampieri, René**
**Le Méditerranée 1 Bd D'Alger**
**F-83600 Frejus (FR)**

(72) Inventeur: **Camus, Patrick**
**315 rue Jean Jaurès**
**F-83600 Frejus (FR)**

**Sampieri, René**
**Le Méditerranée 1 Bd D'Alger**
**F-83600 Frejus (FR)**

(74) Mandataire: **Hautier, Jean-Louis**
**Cabinet Hautier Office Méditerranéen de Brevets**
**d'Invention et de Marques 24 rue Masséna**
**F-06000 Nice (FR)**

(54) **Dispositif pour harmoniser ou réharmoniser l'énergie vitale cosmique chez l'être humain.**

(57) L'invention se présente sous la forme d'un masque reposant sur la face de l'expérimentateur, englobant les yeux et le nez.

L'appareil permet la visualisation d'écrans translucides amovibles colorés (1) en vision mono ou binoculaire par l'intermédiaire de cloison amovible interne (4). Le modèle portatif permet uniquement la vision monoculaire réalisée grâce à la cloison (1). La respiration pour l'appareil s'effectue par l'intermédiaire d'orifices (2) percés dans le masque, un ou plusieurs haut-parleurs (3), solidaires de l'appareil, permettent d'apporter une énergie vibratoire complémentaire à l'aire coloré contenu dans l'appareil. Le devant du masque du modèle portatif comporte une glissière permettant la mise en place de deux demi-écrans opaques (2) ajourés de pastilles colorées translucides (3) dont les couleurs sont appropriées au but recherché. Les demi-écrans (2) mobiles dans leur écartement, permettent la superposition visuelle des pastilles de couleur identique sur chaque demi-écrans par construction mentale binoculaire du phénomène de la vision.

Ce dispositif, apportant le recentrage des yeux, permet à l'énergie cosmique, véhiculée par les pastilles colorées (3), d'être absorbée de manière symétrique par le centre énergétique de chaque oeil.

FIG.2

EP 0 230 393 A1

## Description

La présente invention concerne un dispositif utilisant un procédé qui permet à l'être humain d'harmoniser, ou de réharmoniser, en lui l'énergie vitale à partir de l'énergie cosmique transmise à notre organisme par l'intermédiaire de nos yeux (lumière, couleurs), de nos oreilles (sons), de notre nez (air, prôna), de notre peau (vibrations).

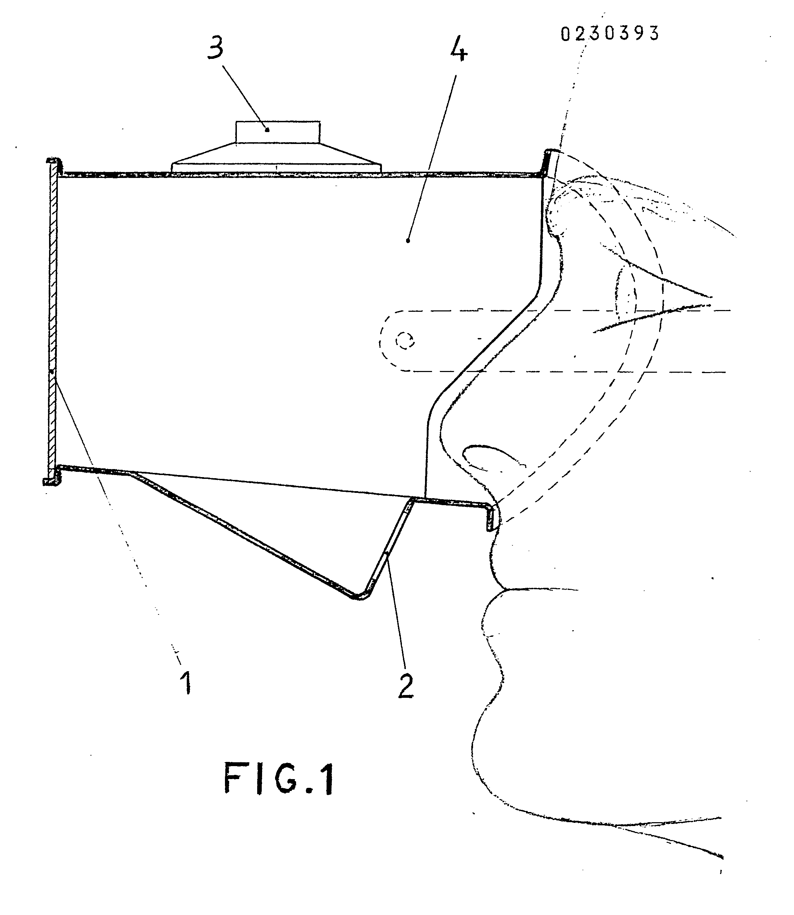

Cette harmonisation, ou réharmonisation, est obtenue par l'intermédiaire d'un masque qui repose sur la face de l'expérimentateur englobant les yeux et le nez. Ce masque (figure 1) aux parois latérales opaques permet la visualisation d'écrans translucides amovibles (1) de couleurs appropriées au but recherché.

Ce masque (figure 1) est percé d'orifices (2) permettant la respiration par le nez de l'air coloré et énergétisé.

Un ou plusieurs haut-parleurs (3) solidaires de l'appareil permettent d'apporter une énergie vibratoire complémentaire à l'aire coloré contenu dans l'appareil.

Le masque (figure 1) comporte un jeu de cloisons amovibles médianes internes (4) permettant la visualisation de couleurs différentes pour chaque oeil et ceci en vision mono ou binoculaire.

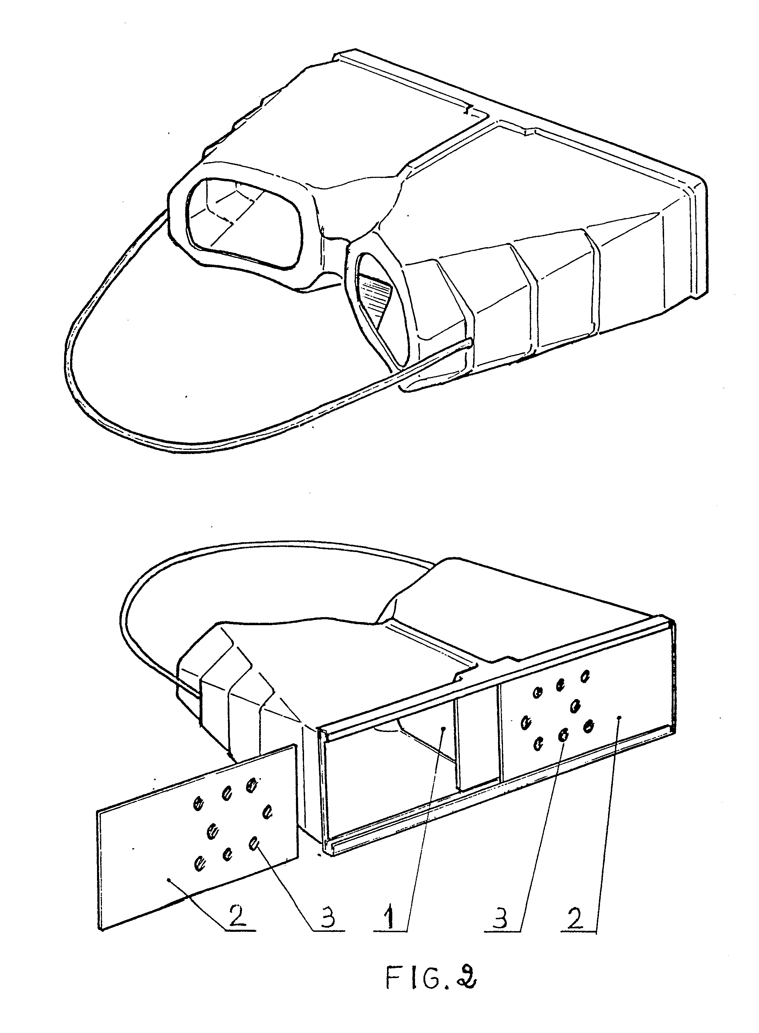

Le dispositif représenté figure 2 concerne le modèle portatif de la présente invention et se caractérise par un masque reposant sur la face de l'expérimentateur, comportant de nouveaux écrans (2), apportant une harmonisation, ou réharmonisation, obtenue uniquement par les yeux.

Ce masque (figure 2) comporte une cloison centrale (1) ne permettant que la vision monoculaire.

Une glissière, sur le devant de l'appareil, permet la mise en place de deux demi-écrans opaques (2) ajourés de pastilles colorées translucides (3) dont les couleurs sont appropriées au but recherché.

Les demi-écrans 2, réglables dans leur écartement, permettent la superposition des pastilles de couleurs identiques de chaque demi-écrans par construction mentale binoculaire propre au phénomène de la vision.

Ce dispositif, apportant le recentrage des yeux, permet à l'énergie cosmique véhiculée et dynamisée par les pastilles colorées d'être absorbée de manière symétrique par le centre énergétique de chaque oeil.

## Revendications

1. Dispositif ayant la forme d'un masque caractérisé par le fait que ce marque reposant sur la face de l'expérimentateur, lui permet d'harmoniser, ou de réharmoniser, en lui l'énergie vitale cosmique par l'intermédiaire de la visualisation d'écrans translucides colorés amovibles au but recherché.

2. Dispositif selon la revendication 1 caractérisé par le fait que la personne respire cet air coloré par des orifices (2).

3. Dispositif selon la revendication 1 caractérisé en ce que l'appareil comporte un ou plusieurs haut-parleurs (3) permettant d'apporter une énergie vibratoire complémentaire à l'air contenu dans l'appareil.

4. Dispositif selon la revendication 1 caractérisé par le fait qu'il comporte des demi-écrans opaques (2) ajourées de pastilles colorées translucides (3) de différentes couleurs permettant d'absorber l'énergie cosmique.

5. Dispositif selon la revendication 1 caractérisé par le fait qu'il comporte une cloison centrale (1) pour que chaque oeil ne perçoive qu'une vision monoculaire.

6. Dispositif selon l'une quelconque des revendications 1 et 4 caractérisé par le fait que les demi-écrans (2) coulissent dans une glissière permettant de régler leur écartement afin que la vision binoculaire se réalise par une construction mentale du cerveau par la superposition de pastilles de couleur identique vue par l'oeil droit et l'oeil gauche.

FIG.1

0230393

FIG. 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | US-A-4 396 259 (MILLER) <br> * Colonne 2, lignes 20-48 * | 1 | A 61 M 21/00 <br> A 61 N 5/00 |
| A | | 4,5,6 | |
| | --- | | |
| Y | WO-A-8 101 509 (FRENKEL) <br> * Page 3, lignes 7-22; page 4, lignes 8-12; page 5, lignes 9-13; figure 2 * | 1 | |
| A | | 2,3 | |
| | --- | | |
| A | CH-A- 455 071 (TOHA) <br><br> * Colonne 1, lignes 1-7; colonne 2, lignes 10-35 * | 1,4,5, 6 | |
| | | | **DOMAINES TECHNIQUES RECHERCHES (Int. Cl.4)** |
| A | US-A-4 388 918 (FILLEY) <br> * Colonne 1, ligne 54 - colonne 2, ligne 9; colonne 4, lignes 3-24 * | 1,3-6 | A 61 M <br> A 61 N |
| | --- | | |
| A | FR-A- 561 229 (AASEN) <br> * Page 1, lignes 29-53 * | 1,2 | |
| | --- | | |
| A | FR-E- 29 863 (GUILHAUMON) <br> * En entier * | 1,3 | |
| | ----- | | |

Le présent rapport de recherche a été établi pour toutes les revendications

| Lieu de la recherche <br> LA HAYE | Date d'achèvement de la recherche <br> 06-04-1987 | Examinateur <br> LEMERCIER D.L.L. |
|---|---|---|